# EUROPEAN PATENT SPECIFICATION

(11) **EP 2 405 828 B1**
(45) Date of publication and mention of the grant of the patent: **25.09.2013**
(21) Application number: 09779125.5
(22) Date of filing: 09.03.2009
(51) Int. Cl.: A61B 17/12, A61B 17/135

(54) **IMPLANT FOR CONTROLLING BLOOD PERFUSION**
IMPLANTAT ZUR KONTROLLE DER DURCHBLUTUNG
IMPLANT DESTINÉ À LA RÉGULATION D'UNE PERFUSION SANGUINE

(43) Date of publication of application: 18.01.2012
(73) Proprietor: AMI Agency for Medical Innovations GmbH, 6800 Feldkirch (AT)
(72) Inventor: REYAL, Julien, A-6800 Feldkirch (AT); EGLE, Walter, A-6842 Koblach (AT)
(74) Representative: Sonn & Partner Patentanwälte
(86) International application number: PCT/EP2009/052731
(87) International publication number: WO 2010/102661

(56) References cited:
- EP-A2- 1 967 168
- WO-A1-89/06513
- WO-A1-98/08466
- WO-A1-2007/142871

## Description

The present invention relates to an implant for controlling blood perfusion within a vessel, especially the portal vein. There is also described the use of such an implant for controlling the blood flow within a vessel and a method for organ transplantation, in particular liver transplantation.

Liver transplantation is the surgical replacement of a diseased liver with a healthy liver allograft. The most commonly used technique is orthotopic transplantation, in which the native liver is removed and the donor organ is placed in the same anatomic location as the original liver. Liver transplantation nowadays is a well accepted treatment option for end-stage liver disease and acute liver failure. Nowadays the one year patient survival rate is about 80-85%, and outcomes continue to improve, although liver transplantation remains a formidable procedure with frequent complications. Unfortunately, the supply of liver allografts from non-living donors is far short of the number of potential recipient's, a reality that has spurred the development of living donor liver transplantation.

Living donor liver transplantation has emerged in recent decades as a critical surgical option for patients with end stage liver disease, such as cirrhosis and/or hepatocellular carcinoma often attributable to long-term alcohol abuse, long-term treated Hepatitis C infection and long-term treated Hepatitis B infection. The concept of living donor liver transplantation is based on the remarkable regenerative capacities of the human liver and the widespread shortage of cadaveric livers for patients awaiting transplant. In this kind of liver transplantation, a piece of healthy liver is surgically removed from a living person and transplanted into a recipient, immediately after the recipient's diseased liver has been entirely removed. The risk of complications in the donor is around 10%, and sometimes a further operation is required. Common problems are biliary fistula, gastric stasis and infections.

In a typical adult living liver transplantation a maximum of 65% of the liver is removed from a healthy living donor. The donor's liver will regenerate to 100% function within 4 to 6 weeks and will reach full volumetric size with recapitulation of the normal structure soon thereafter. Theoretically, it may be possible to remove up to 75% of the liver from a healthy living donor without harming the donor when accepting the risk of an increase of the donor complications, proportional to the increase of graft size.

The transplanted portion will reach full function and the appropriate size in the recipient as well, although it will usually take longer than for the donor.

One of the major problems for the recipient in the course of and after a liver transplantation is excessive portal vein inflow, which may cause allograft dysfunction, in particular in small-for-size living donor liver transplantation. Means to control the blood perfusion in veins are known from US 3,730,186 and US 2006/0129083. However, these means are disadvantageous because they do not allow to monitor the blood flow in the encircled vessel and thus cannot be used to efficiently control blood perfusion.

It is an object of the present invention to provide means to allow the control of blood flow within a vessel, in particular the control of excessive portal vein inflow into an allograft.

WO98/08466 discloses an apparatus for controlling blood perfusion comprising means for measuring the blood flow within a vessel.

The present invention relates to an implant for controlling blood perfusion within a vessel, especially the portal vein, comprising an inflatable band assembly including
a) an non-extendible band configured to be releasably secured around the vessel, wherein the band comprises a tail end and a head end for receiving said tail end and a releasable locking means securing said head and tail ends of said band in an encircling position, and wherein said band includes at least one inflatable balloon arranged at the inner side of the band in its encircling position, said at least one inflatable balloon extending up to 60% over the inner circumference of the band in its encircling position,
b) an injection port with a resealable membrane or a reservoir comprising a fluid, and
c) an inflation tube providing for fluid communication between the injection port or the reservoir and the at least one inflatable balloon of the band assembly, characterised in that at the inner side of the band assembly in its encircling position a means for measuring the blood flow within the vessel is arranged.

The implant of the present invention comprises a non-extendible band with at least one inflatable balloon at its inner side in its encircling position. Said band can be used to encircle any kind of vessel, but is particularly suited to encircle veins and arteries. Bands having a similar assembly are well known to be used as gastric bands. These bands are used to support weight loss in obese patients and are usually made of silicone and placed around the top portion of the stomach, usually via laparoscopic surgery. Such gastric bands are disclosed, for instance, in WO 94/27504, US 2008/0161875, US 2008/0221598 and WO 2005/072195. The closing means releasable securing the non-extendible band around a vessel are also known in the art and are exemplarily described in the documents cited above.

The implant of the present invention further comprises an injection port with a re-sealable membrane which is used to inject a fluid, preferably a solution (like sterile water or a salt solution), in particular an iso-osmolar X-ray contrast solution, to inflate the balloon of the band. Instead of an injection port a reservoir comprising such a fluid can be used. This reservoir is also connected via an inflation tube with the at least one balloon of the band. The reservoir is further connected with a pump which inflates or deflates the balloon of the band depending on the blood flow in the vessel. The fluid to be used for inflation may be a solution, preferably an iso-osmolar X-ray contrast solution, or a gas. The gas preferably is an inert gas or a gas which is not harmful to the human or animal body.

An "iso-osmolar" solution according to the present invention is a saline solution which exhibits substantially the same osmotic pressure as blood or other physiological solutions of the human or animal body and which comprises substances like salts, carbohydrates etc. which are physiologically tolerable or pharmaceutically acceptable.

The inflatable balloon at the inner side of the non-extendible band in its encircling position extends only partly (maximum of 60%, preferably maximum of 50%, more preferably maximum of 40%; minimum of 10%, preferably minimum of 20%, more preferably minimum of 30%, most preferably of 60%) over the inner circumference of the band. If more than one inflatable balloon is provided, the balloons are arranged adjacently at the inner circumference of the band. Due to this assembly the vein is compressed with almost no decrease in its perimeter (the ballon compresses the vein from one side and not circularly) and damages to the vessel can be prevented.

The implant of the present invention further comprises means for measuring blood flow. These means are able to measure the blood flow within a vessel using methods known in the art (e.g. continuous or pulsed wave Doppler methods). Respective methods and means are disclosed for instance in US 5,144,955, EP 0 421 465 and EP 0 938 868.

In order to keep the inner side of the non-extendible band in it encircled position soft enough in order not to damage the encirculated vessel the region adjacent to the at least one inflatable balloon comprises softening means (e.g. silicon) or is connected with the at least one inflatable balloon to allow fluid communication, whereby the thickness of the inflatable balloon is preferably decreased (at least 30%, preferably at least 50%) compared to the region adjacent thereof, so that said region remains substantially undeformed when fluid is introduced in the balloon.

According to a preferred embodiment of the present invention the flow measuring means is a Doppler-type velocity measuring means and/or a transit-time Doppler probe placed next to the band. If the latter means are provided on the band, the band is at least 1 cm wide, preferably 1 to 3 cm, more preferably 1 to 2 cm. The transmit-time Doppler probe which may be positioned around the vessel downstream of the extendible band (direction of the blood flow) is thus an integrative part of the band. Another non invasive possibility of measuring the vessel downstream flow is by perfusion of a magnetic resonance contrast media and simultaneous MRI control (before and just after) the banding adjustments. Furthermore, the blood flow within the vessel can also additionally be measured by an abdominal trans-cutaneous Doppler imaging technique.

According to a further preferred embodiment of the present invention the flow measuring means are positioned opposite to the inflatable balloon.

The non-extendible band of the implant of the present invention is in its encircling position not entirely covered with the inflatable balloon. The flow measuring means are preferably positioned opposed to the inflatable balloon. Advantageously, the flow measuring means are positioned in the vicinity of the locking means and/or the inflatable tube, because said means can be connected to an electric wire which may handicap the use of the implant when the band encircles a vessel and the wire will get into contact with the vessel.

The locking means of the non-extendible band may preferably comprise at least one tooth formed on a portion of the tail end of the band and at least one notch in the head end for engaging said tooth. Alternatively, the locking means of the extendible band comprise at least one barb formed on a portion of the tail end of the band and at least one notch in the head end for engaging said barb.

According to a preferred embodiment of the present invention at least a part of the inflation tube is arranged within a tube having a proximal (or anterior) end and a distal (or posterior) end, the distal end being arranged adjacent to the band assembly.

The inflation tube is at least partly comprised within a tube. The tube allows to easily retrieve the inflation tube and the non-extendible band from the human or animal body after its use.

As used herein, the "posterior" or "anterior" end of the tube is the end part of the tube being positioned towards the outer side of the body when used. Consequently, the "distal" or "posterior" end of the tube is the end part of the tube being positioned to the inner part of the body when used.

In order to retrieve the non-extendible band encircling the vessel means for opening the locking means are preferably comprised within said tube.

The means for opening the locking means are suited to "open" the non-extendible band surrounding a vessel. After its opening the inflation tube as well as the non-extendible band are retrieved from the body of a patient.

According to a preferred embodiment of the present invention the means for opening the locking means comprise a wire adapted to open said locking means, preferably a cutting wire.

In order to open the non-extendible band surrounding the vessel one or more wires may be provided within the tube. The wire may be used to activate a mechanism or to cut the band in order to open the band. According to a preferred embodiment of the present invention the wire is looped around to the locking means.

If the locking means of the non-extendible band comprise, for instance, a barb formed on a portion of the tail end of the band and a notch in the head end for engaging said barb the means for opening the locking means may comprise a cutting wire looped around the barb of the tail end of the band, said wire may further be lead through a flexible (cutting) wire guide (e.g. in the form of a flexible non-extendible tube or spiral spring), before it is connected to a toggle, which will be turned in order to close the loop around the barb, thus cutting through the locking tail of the band. The flexible but non-extendible wire guide functions as a counter bearing, in particular when the wire opens/cuts the band.

In order to protect the non-extendible band and its locking means from **getting attached to fibrotic tissue** the distal end of the tube has preferably a cuff-like form surrounding at least a part or even nearly all of the non-extendible band in its encircling position around a vessel.

According to a preferred embodiment of the present invention the flow measuring means is connected to a transponder and/or an electric pumping implant for pumping a fluid, in particular a solution, from the reservoir to the inflatable balloon of the band and vice-versa.

The flow measuring means of the implant according to the present invention are preferably connected via electric wires to a transponder. This transponder, which may be positioned in the vicinity of the injection port or reservoir, transmits the data collected with the flow measuring means to a receiving unit outside the body. These data can be monitored outside the body and used to control and - if required - to change the portal vein flow by inflating or deflating the inflatable balloon of the non-extendible band. The flow measuring means may also be connected to en electric pumping implant. This pumping implant may be connected via an inflation tube to a reservoir which comprises an iso-osmolar solution. The pumping implant may further comprise a central processing unit controlling a pump to influence the blood perfusion within a vessel by automatically inflating or deflating the inflatable balloon of the non-extendible band.

The band assembly may not only comprise means for measuring blood perfusion but also for measuring the pressure within the vessel. A pressure measuring means is therefore preferably arranged at the inner side of the band in its encircling position.

Of course it is also possible to use a second band encircling the vessel which only comprises means for measuring the pressure within a vessel.

According to a preferred embodiment of the present invention the proximal end or anterior end of the tube (opposite to the end, where the band assembly is positioned) is sealed with a sealing means, preferably a (thin) membrane, in order to protect bowel and fibrotic tissue entering the lumen of the tube.

The sealing means is preferably a self-sealing elastomer, preferably a silicon or polyurethane plug, more preferably a removable silicon or polyurethane plug.

The implant according to the present invention is preferably provided as a disposable product.

The band, the inflating tube and/or the tube are preferably flexible. Therefore these components of the implant of the present invention are preferably made from an elastomer, preferably from silicone.

According to a preferred embodiment of the present invention the inflation tube and the tube comprising the inflation tube are of the same or similar material or of a material having similar characteristics as the inflatable band.

The inner diameter of the band in its encircling position is preferably between 0.8 to 3 cm, preferably 1 to 2 cm.

The non-extendible band has preferably a length of 2 to 8 cm, preferably of 2.5 to 6 cm.

According to a preferred embodiment of the present invention the non-extendible band has a width of 0.5 to 3 cm, preferably 0.7 to 2.5 cm, more preferably 1 to 2 cm.

The tube comprising the inflatable tube has an inner diameter in between 1 to 4 cm, preferably in between 1.5 to 3.5 cm, more preferably in between 2 to 3 cm. The diameter can be varied depending on the tubes and wires provided in the implant of the present invention.

According to a further preferred embodiment of the present invention the tube has a length of 5 to 40 cm, preferably of 10 to 30 cm, more preferably of 10 to 25 cm.

The non-extendible band assembly with the inflatable balloon enables reduction of blood perfusion in the vessel in the range of 0 to 100%, preferably 0 to 90%, more preferably 0 to 80%, without negatively affecting the wall of the encircled vessel.

In some cases a reduction of the blood flow to 0% is not required, because this can be a negative impact (e.g. on the liver allograft regeneration) and can be dangerous to the upstream venous pressure (e.g. portal venous pressure in liver transplantation). However, if needed for a very short term, the total stop of blood flow by completely closing the band, would not be harmful to the encircled vessel, due to the specific assembly of the band (the inflatable balloon extends only up to 60% on the inner side of the band).

The use of an implant according to the present invention for controlling the blood flow in a vessel in the course of and/or after surgery is also described, preferably in the course of and/or after an allograft transplantation, in particular in the course of and/or after liver transplantation.

The implant of the present invention can be used to control blood perfusion in vessels, in particular in veins, before, during and after surgery. Therefore the implant can be used for various surgical interventions like transplantations. In particular organ transplantations require to reduce or even to impede blood perfusion towards and away from the organ to be transplanted.

There is further described a method for performing an organ transplantation, especially an at least partial liver transplantation, comprising the step of controlling blood perfusion within a vessel with an implant according to the present invention.

According to a preferred embodiment of the present invention the blood perfusion of at least one vessel leading to an organ to be transplanted is controlled prior the organ retrieval from the donor and/or the transplantation of the organ into the recipient. The blood perfusion of at least one vessel, in particular one vein, leading to the organ to be transplanted may be reduced with the implant of the present invention.

A method of implanting and retrieving an implant according to the present invention is also described comprising the steps of introducing a tube comprising an inflating tube and an non-extendible band into a human or animal body and encircling a vessel of interest, performing the surgical intervention and removing the non-extendible band surrounding the vessel by using means for opening the locking means.

There is also described a method for controlling blood perfusion within the portal vein in the course of and/or after liver surgery comprising the steps of:
- opening the abdominal wall,
- dissecting the portal vein,
- placing a non-extendible band of an implant according to the present invention around the dissected vein,
- reducing the blood flow by inflating the inflatable balloon of the band until the desired level of portal flow is achieved, measured intra-operatively and accordingly to the future remnant liver size as identified by pre-operative imaging.
- dissecting and removing the affected section(s) of the liver
- closing the abdominal wall and
- progressively deflating the banding over a period of at least 10 days from surgery.

There is further described a method for controlling blood perfusion within a vessel in the course of and/or after liver transplantation comprising the steps of:
- providing a liver or partial liver graft thereof from a donor,
- opening the abdominal wall of the recipient,
- clamping the portal vein and removing the diseased liver from the recipient
- the opened band of the present invention around the portal vein preferably upstream to the clamp,
- putting the graft in the abdominal cavity of the recipient,
- anastomosing of the sushepatic vein and the portal vein
- the sushepatic and/or portal vein clamp,
- closing the band around the portal vein,
- completely filling the balloon of the band
- deflating the balloon progressively until the lumen of the band provides the desired flow rate (depending upon the graft size) of the portal vein,
- anastomosing the hepatic arterial and biliary tract,
- closing the abdominal wall is closed, and
- progressively deflating the band over a period of at least 5 days after surgery, preferably at least 10 days (preferably at least 5 up to 20 days, more preferably up to 14 days, even more preferably up to 10 days).

The method can be used for any liver transplantation, however it is particularly useful for living donor liver transplantation. The technique may involve the creation of a temporary left portahepatic shunt. This allows portosystemic decompression during implantation of the donor liver and diverts mesenteric blood from the donor liver during the crucial period unhepatic period.

The implant of the present invention can further be used for various surgical interventions. The implant can be used in hepatectomy (up to 90% resection) in normal liver patients, or also for hepatectomies in cirrhotic patients, where these interventions would not be possible without banding leading to an increased survival rate of the patients treated. The banding further reduces the risk of liver small-for-size syndrome by initial protection of the liver and its sinusoidal endothelial cells (SEC) against a too high portal vein inflow, and control of an ideal regeneration trigger by just one fold increase of the portal flow post-resection or post-transplantation. Banding also allows a controlled increase of the portal inflow, harmoniously to the physiologic liver regeneration and liver weight increase, particularly simultaneously with the endothelial sinusoidal cell (SEC)regeneration timing resulting in stimulation of the SEC regeneration, and liver global regeneration, as a second regeneration trigger at the moment of banding deflation.

The implant can also be used in living donor liver transplantation and decease donor liver transplantation. This makes it possible to transplant more patients in need of a transplantation, by reducing the shortage of liver graft, thanks to systematic split of decease donor livers in two or three splitted grafts, or ethically acceptable living donor left lateral segment retrieval by laparoscopy or laparotomy. Banding allows to use very small liver graft, approximately 15%-20% of total liver weight, or a graft recipient weight ratio (GRWR) between 0,3 to 0,5%, which would not be possible without banding. Furthermore, in case of living donor the retrieval of only the left lateral segment is sufficient instead of a much more dangerous right liver retrieval, with a significant morbidity leading to a reduction of the morbidity and no more mortality of the donor without increase of the morbidity and mortality of the recipient.

The present invention is further illustrated in the following figures and examples without being restricted thereto.

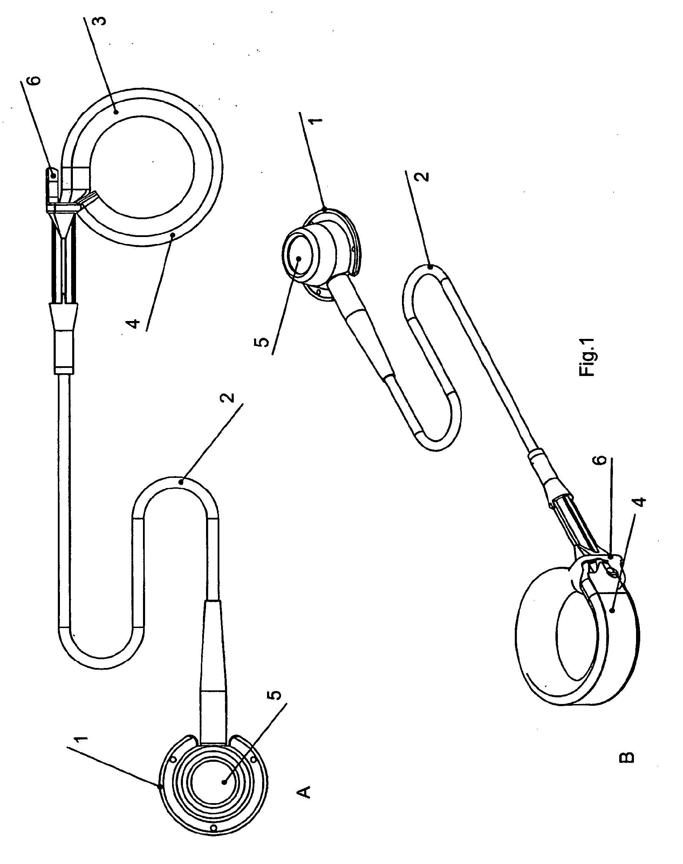

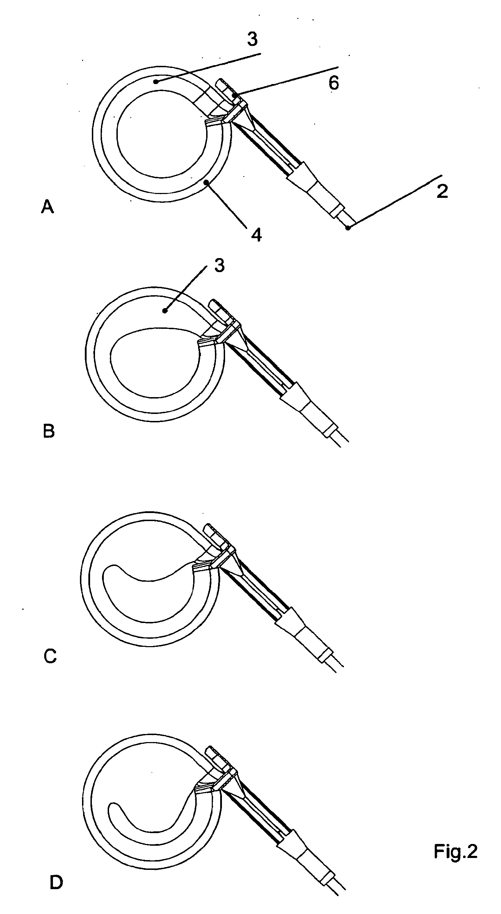

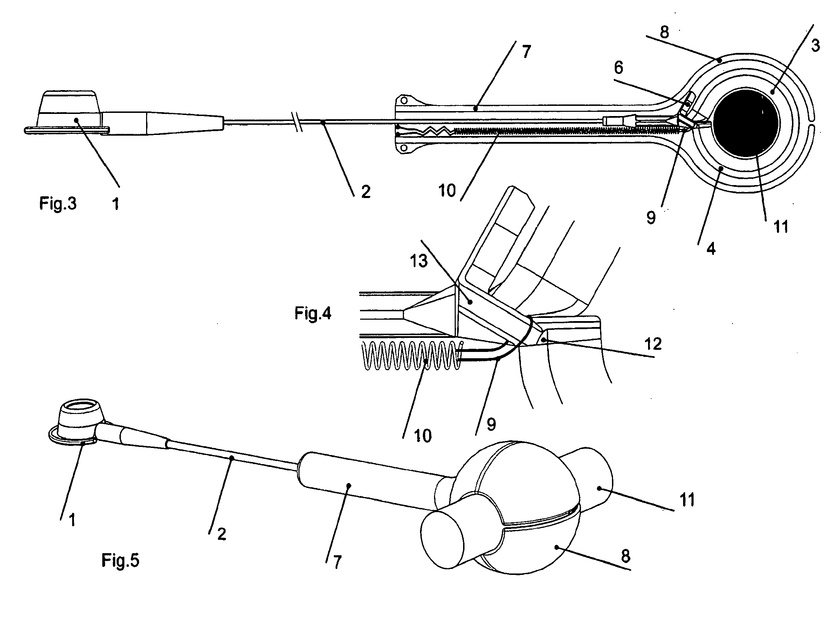

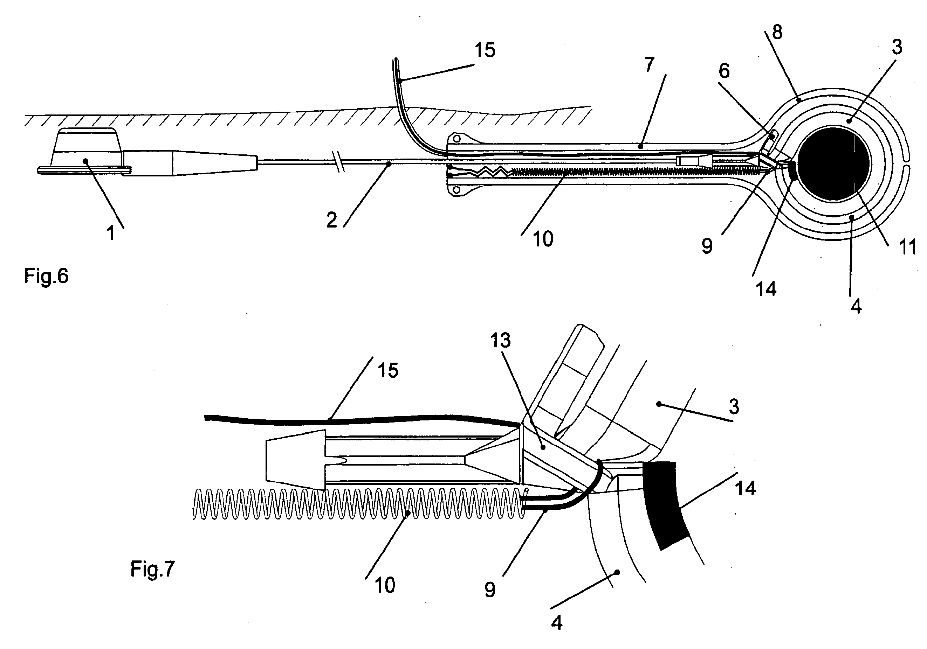

Fig. 1 shows two views of an implant according to the present invention; Fig. 2 shows different inflation levels of the band assembly; Fig. 3 shows a sectional view of a band assembly according to the invention comprised within a tube; Fig. 4 shows locking means of the bond assembly; Fig. 5 shows a band assembly within a tube where the distal end has a cuff-like form; Fig. 6 shows a sectional view of a band assembly of the present invention; Fig. 7 shows an enlarged view of the locking means of the band assembly.

Fig. 1 A and B show an implant of the present invention comprising an injection port 1 connected via an inflation tube 2 to an inflatable balloon 3 of an non-extendible band 4. The injection port 1 comprises further a resealable membrane 5. The resealable membrane 5 forms a sterile barrier, which is impermeable to any kind of fluid. This membrane 5, however, can be penetrated by a syringe tip in order to bring an iso-osmolar solution into and out of the system to inflate or to deflate, respectively, the balloon 3. The resealable membrane 5 may comprise an elastomer.

The non-extendible band comprises further releasable locking means 6. These means allow to bring the band 4 in an encircled position around a vessel and to prevent the opening of the non-extendible band during inflation and deflation of the inflatable balloon 3. Since these locking means are releasably fixed they can be opened easily after the use of the band.

Figs. 2 A to D show an non-extendible band 4 in various stages of inflation. Fig. 2A shows a deflated inflatable balloon 3. Since the inflatable balloon 3 does not extend completely over the entire inner circumference of the non-extendible band 4 in its encircled position the inflation of the balloon 3 does substantially not affect the perimeter of the inner side of the encircled band. In order to keep the inner side of the non-extendible band in it encircled position soft enough in order not to damage the encirculated vessel the region 16 adjacent to the inflatable balloon 3 comprises softening means (e.g. silicon) or is connected with the inflatable balloon 3 to allow fluid communication, whereby the thickness of the inflatable balloon 3 is decreased (at least 30%, preferably at least 50%) compared to the region 16 adjacent thereof, so that said region 16 remains substantially undeformed when fluid is introduced in the balloon 3.

Fig. 3 shows an implant of the present invention which components are partly comprised within a tube 7 having a proximal end and a distal end. The distal end 8 of the tube 7 has a cuff-like form. This form allows to surround at least a part of the non-extendible band 4 in its encircling position around a vessel 11.

The tube 7 further comprises a wire 9 arranged within a wire guide 10 which is used to open/release locking means 6.

Fig. 4 shows locking means 6 securing the non-extendible band 4 in an encircling position. The locking means 6 comprise a tooth 13 formed on a portion of the tail end of the band 4 and a notch 12 in the head end for engaging said tooth 13. A wire 9 arranged within a wire guide 10 surrounds tooth 13. The application of a force to the wire guide 10 and wire 9 results in an opening of the band 4.

Fig. 5 shows an implant of the present invention comprising a tube 7 whose distal end 8 has a cuff-like form. The implant is positioned around a vessel 11.

Fig. 6 shows an implant of the present invention which comprises at the inner side of the band assembly in its encircling position a flow measuring means 14. Said flow measuring means 14 is connected to a wire 15 comprised within a tube 7. Information obtained by the flow measuring means is transmitted by the wire 15 directly or via a transponder to a central processing unit. The central processing unit allows to visualise the data obtained by the flow measuring means.

Fig. 7 shows an enlarged view of the locking means of the band assembly as depicted in Fig. 6.

The implant of the present invention can be used exemplarily for the following surgical interventions.

### 1) Liver surgery (hepatectomy)

In this example a liver surgery is described, wherein from 50% to 90% of the total liver weight in case of non-cirrhotic patient and liver resection in case of cirrhotic patient was removed. In the course and after surgery a banding was put around the patient portal vein. The surgery involved the following steps:
- Dissection of the main trunk of the portal vein
- Banding is preferably placed at the base (caudal) of the dissected vein
- Portal pressures is preferably measured continuously (up and down of the banding, by a Swan-Ganz catheter introduced in a little branch of the portal vein system, like jejunal branch)
- Downstream portal flow is preferably measured continuously by perivascular flow probe (transit-time flow measurement)
- Dissection of the portal vein branch of the segments to be retrieved
- Measurement of the flow of the segment portal branch to be retrieved
- The portal vein flow of the non-retrieved segment is calculated by subtracting the retrieved portal branch flow from the main trunk flow
- Alternatively the portal vein flow of the retrieved segment could be calculated as follows: (100 - the CT-scan preoperative assessment of the liver percentage to be retrieved) x main trunk portal vein flow (for example, a 80 % liver resection results in (100-80) x main trunk flow
- Portography by the Swan-Ganz catheter can optionally be conducted in order to check for big portosystemic collaterals
- optionally the hepatic artery flow can be measured by transit time flow measurement (TTFM)

Before parenchymal transsection the portal vein flow was set to 2x, 2.5x or 3x of the initial non-retrieved portal vein branch flow, when non-cirrhotic liver was dissected, and to 1x, 1.5x and 2x of the initial non-retrieved portal vein branch flow, when cirrhotic liver was dissected.

In case of an increase of the upstream pressure in cirrhotic patients with collateral circulation, the coronary varicose veins were ligated to avoid varicosal bleeding and/or portal thrombosis. Instead of a ligation the coronary varicose veins can be occluded intravascularly by coils during portography, or the oesophageal varice can be ligated by endoscopy during the surgery or after the surgery.

After parenchymal transsection and at the end of the hepatectomy the downstream pressure and flow was controlled and, if necessary, the portal downstream flow was adjusted by banding. After the reduction of the liver mass, the downstream portal pressure may be increased.

In case of major portosystemic shunting(PSS), a further banding (PSSB) can be put around the PSS after dissection in order to postoperatively control the portal flow by closing or reopening the PSSB if needed and to avoid portal steal flow. This can be assessed by Doppler or perivascular flow probe, which can be left in place after abdominal wall closure.

The catheters and flow probes are removed, but the flow probe can be left in place in order to postoperatively control the portal inflow.

After closing the abdominal wall the banding implant of the present invention encircling the portal vein can be used to further control the blood flow through said vein. Usually on days 1 to 7, preferably on days 1 to 5, more preferably on days 1 to 3, the inflation is kept substantially identical as in the course of the surgery.

On days 3 to 15, preferably days 5 to 15, more preferably days 5 to 7, after surgery the inflatable chamber of the portal vein band is progressively or immediately deflated. If the deflation is performed progressively the deflation may occur stepwise, for instance, from 70% inflation to 0% inflation, in one, two or three deflations.

The deflation of the vein band can follow a defined deflation algorithm calculated from the relation between inflation-deflation, flow-pressure variations, and liver regeneration speed, and/or by measuring the periportal flow.

The healing progress can be observed by liver function tests, portal vein transit-time Doppler probe and portal vein echography, CT-Scan at 1, 2, 3, 4, 5, 7, 10, 12 or 14 days after surgery.

After complete deflation of the banding the injection port or the reservoir can be removed from the recipient's body. A removal may occur after a minimum of 8, preferably of 10, more preferably of 12, in particular of 14, days after surgery. This intervention can be done under local anaesthesia. The tubes and the inflatable band and optionally the PSSB and periportal flow probe can be removed together with the injection port or the reservoir or up to 1 year after surgery by coelioscopy, laparotomy or with the retrieval system described herein.

### 2) Living donor liver transplantation

In the course of living donor liver transplantation the non-extendible band of the present invention can be used to control blood perfusion of the portal vein of the recipient as well as of the donor.

The left lateral segment of the liver (segment 2 and 3) is retrieved from the donor. Prior the organ retrieval the portal vein flow arterial flow of the retrieved liver segment is preferably measured by TTFM preferably after dissection of the left pedicle elements, and parenchymal transsection, and before vascular sections.

Preoperatively the major portosystemic collaterals of the recipient are assessed by CT-Scan or MRI:
- dissection of the liver ligaments
- dissection of the vascular elements (portal vein, hepatic artery, biliary duct, hepatic veins)
- section of the hepatic artery, biliary duct
- before portal and sushepatic veins sections, introduction of a Swan-Ganz catheter in a jejunal vein, for measurement of the main trunk portal pressure upstream and downstream to the banding
- measurement of the main trunk portal flow by perivascular transit-time flow probe
- measurement of the Portal perimeter at the future banding location
- portography by the jejunal catheter, to see the collateral veins, particularly the coronary varicose veins, and major portosystemic collaterals like splenorenal shunt. The transplantation may comprise the following steps:
- the band of the present invention is put around the portal vein of the recipient according to the measured portal vein perimeter
- portal clamp
- portal and sushepatic veins sections
- retrieval of the recipient's liver
- fixation of the tube on the injection port or reservoir, after passing through the lateral abdominal wall
- the injection port or reservoir is preferably placed sub-cutaneously
- the band is deflated during the transplantation (there is a portal clamp down to the banding)
- if a portocaval shunt is made to decompress the portal upstream, the portocaval shunt flow is measured by a transit-time flow probe to assess the flow capacity of the portal system after liver transplantation completion, and at the banding deflation (portal flow capacity=portocaval shunt flow); if no portocaval shunt is constructed, a PVC vascular shunt tube is inserted between the portal vein end, and a lateral hole created in the inferior caval vein (IVC), and sutured by a purchase string suture at the IVC wall, and a vascular tourniquet around the portal vein and the tube shunt
if the portal capacity flow is inferior to 200 ml/min 100gr transplanted graft weight, the portosystemic collaterals detected by preoperative RMI or CT-Scan, or intraoperative portography
- are ligated intraoperatively, or intravascularily occluded; first, the small collateral, and if not sufficient, the major collaterals, from the smallest to the biggest; if the portal vein is thrombosed, a thrombectomy is made; if the portal vein is too narrowed, a surgical bypass is made between the mesenteric superior vein, or left renal vein, or a cavo-portal transposition is created.
- if the portal capacity flow is inferior to 1000 ml/min, the major portosystemic collaterals are reconstructed and the biggest collateral is dissected, and a second banding (PSSB PortoSystemic Shunt Banding) is put around this major collateral to control post-operatively the portal graft flow after 5 days, by closing the PSSB, and avoiding a portal steal flow syndrome, without peroperative sudden increase the portal pressure upstream to the portal banding
- the PSSB can be reopened postoperatively after closing; high portal pressure can induce complications
- PSSB is passed through the abdominal wall and the injection port or reservoir is fixed like the portal banding, at a different location.
- the PSSB is occluded to assess the increase of portocaval shunt flow superior to 1000 ml/min
- thereafter the PSSB is let not inflated
- if required a second PSSB is installed in the same manner
- introduction of the graft
- hepatic vein anastomosis
- portal anastomosis between left donor portal vein and preferably recipient left portal vein or main trunk portal vein
- the inflatable balloon of the non-extendible band is preferably completely inflated (100%)
- a TTFM probe is put around the donor portal vein
- the sus-hepatic vein clamp is first removed, followed by the removal of the and portal vein clamp
- continuous measurement of the portal pressure upstream (jejunal vein Swan-Ganz catheter), and downstream pressure
- continuous measurement of the portal downstream inflow
- hepatic vein clamp removed
- progressive deflation of the balloon of the band,e.g. 0.01 ml + 0.01ml etc...
- permanent control of the downstream pressure and flow during the deflation
- the downstream values are maximum donor in-vivo graft flow x2 or 2.5 or 3, and a portal downstream pressure inferior to 15 mmHg
- in case of pre-existent portal hypertension, the upstream portal pressure will be respected as far as possible, to always have the same pressure
- in case of the upstream pressure increase in cirrhotic patients with collateral circulation, the coronary varicose veins will be ligated to avoid varicosal bleeding and/or portal thrombosis, or instead of ligation they will be occluded intravascularly by coils during portography, or the oesophageal varice will be ligated by endoscopy during the surgery or after the surgery
- the catheters are removed
- the flow probe is removed, or let in place if necessary
- arterial anastomosis
- biliary anastomosis (anse-en-Y de Roux)
- abdominal wall closure

After completion of the transplantation the inflation level of the non-extendible band will be controlled (post-operative use of the implant of the present invention). At day 1 to days 2 to 7, preferably 3 to 5, no modification of the inflation level will occur.

After day 2, preferably between days 3 to 15, more preferably between days 5 to 10 the balloon of the band will be progressively or stepwise deflated (ideally from 70% inflation to 0% inflation, in at least one or two deflation steps).

If a PSSB was installed, the PSSB can be deflated at the same day as the portal banding to increase the portal flow, and make it sufficient to a perfect liver regeneration. The PSSB manipulation is assessed ideally by periportal flow probe.

The deflation of the vein band can follow a defined deflation algorithm calculated from the relation between inflation-deflation, flow-pressure variations, and liver regeneration speed, and/or by measuring the periportal flow.

The healing progress can be observed by liver function tests, portal vein transit-time Doppler probe, portal vein echography, CT-Scan or best by repeated MRI for simultaneous portal flow and graft regeneration measurement at 1, 2, 3, 4, 5, 7, 10, 12 or 14 days after surgery.

After complete deflation of the banding the injection port or the reservoir can be removed from the recipient's body. A removal may occur after a minimum of 8, preferably of 10, more preferably of 12, in particular of 14, days after surgery. This intervention can be done under local anaesthesia. The tubes and the inflatable band and optionally the PSSB and periportal flow probe can be removed together with the injection port or the reservoir or up to 1 year after surgery by coelioscopy, laparotomy or with the retrieval system described herein.

### 3) Decease donor liver transplantation

In decease donor liver transplantation the banding may be put in the recipient or donor portal vein after portal vein anastomosis.

The recipient is prepared for surgery as described above.

The donor liver is preferably split and retrieved as follows, whereby all of the steps are performed on a beating heart patient:
- after classical preparation and dissection of the donor organs
- in-situ-split of the left lateral segment
- the liver anatomy is known by preoperative CTScan and RMI assessment (particularly the segmental volume, and portal, arterial, and biliary anatomy)
- after left pedicular dissection, and before parenchymal transection, the liver surgeon measure the portal blood flow of the main trunk, the left portal branch, and the right portal anterior and posterior branch, with a transit-time flow probe , or just the left branch
- attention to the arterial branch of the Segment 4 of the liver: the section of the left hepatic artery is ideally made downstream to the branch
- parenchymal transection of the left lateral segment(LLS)
- retrieval of the LLS after vascular sections (portal left vein, left artery, left hepatic vein)
- cold storage in a preservation solution, like University of Wisconsin Solution at 4°C on a back-table
- preparation of the right liver split by hanging manoeuvre between right and middle hepatic veins, and right anterior and posterior portal veins
- classic cold storage of the body and liver by the aortic cannula and the portal vein (after the heart has just been retrieved by cardiac surgeon)
- retrieval of the remnant liver (segment 1-4-5-6-7-8) with classic fashion
- cold storage at 4°C with preservation solution, by portal vein and the segment 4 portal vein orifice
- for the LLS, the backtable time is very fast,
- preparation of the anastomosis, hepatic left vein enlargement (reconstruction) if necessary
- if transplantation of the all right liver in one part, classical back-table of a cadaveric liver transplantation (caval vein preparation, pedicle examination, arterial reconstruction if necessary, like two right hepatic arteries)
- if decision of split of the liver in two part (according to portal anatomy) the split of the right liver may be in situ during cadaveric beating heart retrieval
- dissection of the right anterior and posterior pedicle
- if not realized during cadaveric retrieval, liver hanging manoeuvre, which permits to place a loop between the right and the middle hepatic vein
- transaction of the liver between the anterior and posterior sectors
- the caval vein is let to the anterior graft (1-4-5-8 segments) or can be let with the posterior graft (segment 6-7)
- the main trunk of portal vein, hepatic artery and biliary duct are let to the anterior segment
- reconstruction of the segment 4 portal vein if necessary, with donor vessels
- reconstruction of the hepatic veins of segment 5 and segment. 4 if needed with donor vessels
- the two liver grafts are ready for the transplantation in two adults recipient's
- during the time of the back-table, the recipient's liver has been retrieved

### 4) Liver transplantation, particularly partial graft (living donor or decease donor), banding around a major(s) portosystemic(s) collateral(s), to control the portosystemic flow, and to prevent the portal steal syndrome, without the application of a portal vein banding

Without use of the concept of dynamic portal banding (with use of portal banding is described above):
- during the reflow after portal vein anastomosis, if the portal flow of the graft is inferior to 1000 ml/min, or inferior to 200ml/min per 100 gr graft weight(in case of partial graft), the portal steal flow is expected. So a major PSS is prepared, dissected, and a PSSB is put around. This PSSB is inflated progressively in post-operative period, to avoid portal pressure increase, by closure of the PSS, after preferably 3 to 5 days, assessed by periportal flow probe, or Doppler.
- the PSSB without portal banding can be used in two situations:
- preventive: in case of preoperative assessment of major PSS, a PSSB can be systematically put around the biggest PSS, to

control the portal flow post-operatively, and to avoid portal steal flow by the PSS, in case of Doppler or periportal flow probe postoperative assessment of portal flow decrease under 1000 ml/min
- therapeutic: in case of preoperative reflow portal flow inferior to 1000 ml/min, or 200ml/min per100 gr graft weight, to permit to close the PSS post-operatively, or intraoperatively, but with the possibility to open the PSSB in case of postoperative liver function decrease (small-for-size graft syndrome)
- the PSSB can be deinflated postoperatively, after inflation, in case of too high portal pressure, revealed by portal hypertension complications, like varicosal bleeding.

### 5) Auxiliary orthotopic or heterotopic partial graft liver transplantation, with banding around the portal vein of the auxiliary graft, or around the portal vein of the remnant liver

In case of auxiliary transplantation (for specific indications, like fulminant liver failure) orthotopic (after removal of the half of the native liver, and put the partial graft instead of the retrieved native liver) or heterotopic (native liver left in place, and graft put in another place) the concept of dynamic portal vein banding to permit a control of the graft portal flow, and the perfect graft regeneration because of the ideal inflow can be applied.
A) In case of orthotopic auxiliary transplantation, and banding put around the portal vein of the native liver:
   - the graft portal flow is set at the value of preferably 2.5 x the donor graft portal flow
   - post-operatively, preferably at day 3 or 5 when the native liver function amelioration start (assessment by native liver Tc99m-GSA galactosyl serum albumin scintigraphy one time before transplantation, and regularly after transplantation)
   - the banding is progressively inflated, to increase the graft portal flow at his maximum
   - when the liver function is sufficient to support a new surgery, and when the native liver portal flow is reduced to zero, the sick native liver is removed with the banding
   - the surveillance of the banding is the same as described above
B) In case of orthotopic auxiliary transplantation, and banding put around the portal vein of the graft:
   - the portal flow of the graft is controlled at 2.5 x the donor portal flow
   - postoperatively, preferably at day 3 or 5, the banding is progressively inflated, to increase the portal flow of the native liver, and permit a perfect regeneration of the native liver let in place (in case of reversible state, like fulminant liver failure)
   - when the portal flow of the native liver is maximal, the graft is removed with the banding
C) Heterotopic auxiliary transplantation, with banding put around the graft portal vein:
   - the portal flow is controlled at the ideal flow
   - postoperatively, the banding is progressively deflated or inflated, to increase the graft portal flow
   - the graft is removed with the banding, when the liver function of the native liver is back to normal
D) During all of those auxiliary transplantation procedures, if needed, the PSSB can be used, with the same protocol as described above

### 6) Pringle manoeuvre during liver surgery, banding put around the hepatic pedicle

- the portal banding can be used around the hepatic pedicle, during hepatic resection surgery, to realize a Pringle manoeuvre, with an atraumatic soft technique.

### 7) Remote Pringle manoeuvre during major liver trauma, banding put around the hepatic pedicle, before interventional radiology

- the portal banding can be used around the hepatic pedicle, or the hepatic artery after dissection of the pedicle, or the portal vein after dissection
- in case of hemorrhagic liver trauma, in a emergency procedure the application of the portal banding during laparotomy, as a pringle manoeuvre, permit to realize an removal control of the hemorrhage, to avoid lethal hemorrhagic shock. After that, the port is installed under the skin, or through the skin, the liver is packed, the wall is closed, and the patient is transported to the interventional radiology room, to make a hepatic arteriography. The banding is completely deflated at the start of the arteriography to permit it.

### 8) All type of portosystemic shunt (portocaval shunt too) congenital, surgical, or spontaneously acquired, banding put around the shunt

- in all cases of portosystemic shunt, the portal banding can be used to treat this pathology, and to avoid sudden increase of the portal pressure and its complications due to sudden closure of a shunt.
- the banding is put around the shunt during the surgery after dissection of the shunt
- the banding is progressively inflated postoperatively, during several days or weeks, to reduce to internal diameter of the shunt, and decrease the symptoms of the patients (like hepatic encephalopathy, or others complications of a portosystemic shunt)
- the banding is in a second step removed by surgery, like the portal banding removal technique (laparotomy, or laparoscopy, or use of the easy retrieval system)

### 9) Veterinary surgery, intrahepatic or extrahepatic portosystemic shunt in dog or cat, banding put around the portal branch that irrigate the intrahepatic shunt, or around the shunt itself if extrahepatic.

- in case of veterinary pathology, the portosystemic shunt is a very common pathology, in cat or dog, with a high morbidity and mortality without treatment.
   A) In case of intrahepatic portosystemic shunt (assessment by CT-Scan):
      - the portal vein that irrigate the PSS is dissected, and the banding is put around this branch of the portal vein.
      - postoperatively, the banding is progressively inflated to reduce the flow of the shunt, and passively close it,in several days or weeks
      - the banding is removed in a second step
   B) In case of extrahepatic portosystemic shunt, the banding is put around the dissected shunt, and progressively closed post-operatively during few days, or weeks, to close the shunt, and decrease all the symptoms of the PSS.

In the course of a liver transplantation the inflatable band can be put around the portal vein of the recipient after total liver retrieval. A tube comprising the inflation tube is passed through the abdominal muscles (up or down to the bi-subcostal incision). The inflation tube is connected with the injection port or with a reservoir comprising an iso-osmolar liquid. A tunnel can be created between the skin and the fascia layer to permit to place the injection port without another skin incision, and to avoid infection of the injection port during the post-operative deflation of the banding. The port can be fixed to the fascia plan. The banding is completely closed just before the portal clamp removal to avoid hyperperfusion liver lesions.

The injection port is preferably positioned on the right basithoracic wall, because the thoracic wall is more rigid it does not move like the abdominal wall. Furthermore, after liver transplantation many drains and tubes are positioned through the abdominal wall and the thoracic wall is free of such tubes.

The inflation tube or the tube comprising said inflation tube may be positioned between the first liver and duodenum and between liver and curvature of right colon. This route is the shortest route between the port and the portal vein.

## Claims

1. Implant for controlling blood perfusion within a vessel (11), especially the portal vein, comprising an inflatable band assembly including
a) an non-extendible band (4) configured to be releasably secured around the vessel (11), wherein the band (4) comprises a tail end and a head end for receiving said tail end and a releasable locking means (6) securing said head and tail ends of said band (4) in an encircling position, and wherein said band (4) includes at least one inflatable balloon (3) arranged at the inner side of the band in its encircling position, said at least one inflatable balloon (3) extending up to 60% over the inner circumference of the band (4) in its encircling position,
b) an injection port (1) with a resealable membrane (5) or a reservoir comprising a fluid, and
c) an inflation tube (2) providing for fluid communication between the injection port (1) or the reservoir and the at least one inflatable balloon (3) of the band assembly, wherein in that at the inner side of the band assembly in its encircling position a means for measuring the blood flow (14) within the vessel (11) is arranged and wherein at least a part of the inflation tube (2) is arranged within a tube (7) having a proximal end and a distal end (8), the distal end (8) being arranged adjacent to the band assembly, said distal end (8) of the tube having a cuff-like form surrounding the non-extendible band (4) in its encircling position around the vessel (11), wherein means for opening the locking means (6) are comprised within said tube (7) which comprise a wire (9) adapted to open said locking means, preferably a cutting wire, wherein the wire (9) is looped around to the locking means (6), in particular looped around at least one tooth or barb (13) of a tail end of the band (4) comprised within the locking means (6) further comprising at least one notch (12) in the head end for engaging said tooth (13) or barb, said wire (9) being arranged within a flexible non-extendible wire guide (10) fixed on the tube (7).

2. Implant according to claim 1, **characterised in that** the flow measuring means (14) is a Doppler-type velocity measuring means, and/or a transit-time Doppler probe placed downstream to the band.

3. Implant according to claim 1 or 2, **characterised in that** the flow measuring means (14) are positioned opposite to the inflatable balloon (3).

4. Implant according to any one of claims 1 to 3, **characterised in that** the locking means (6) comprise at least one tooth (13) or a barb formed on a portion of the tail end of the band and at least one notch (12) in the head end for engaging said tooth (13) or barb.

5. Implant according to any one of claims 1 to 4, **characterised in that** the flow measuring means (14) is connected to a transponder and/or an electric pumping implant for pumping fluid from the reservoir to the inflatable balloon (3) of the band and vice-versa.

6. Implant according to any one of claims 1 to 5, **characterised in that** a pressure measuring means is arranged at the inner side of the band (4) in its encircling position.

7. Implant according to any one of claims 1 to 6, **characterised in that** the proximal end of the tube (7) is sealed with a sealing means, preferably a membrane.

8. Implant according to claim 7, **characterised in that** the sealing means is a self-sealing elastomer, preferably a silicon or polyurethane plug, more preferably a removable silicon or polyurethane plug.

9. Implant according to any one of claims 1 to 8, **characterised in that** the band (4), the inflating tube (2) and/or the tube (7) is made from an elastomer, preferably from silicone.

10. Implant according to any one of claims 1 to 9, **characterised in that** the inner diameter of the band (4) in its encircling position is between 0.8 to 3 cm, preferably 1 to 2 cm.

11. Implant according to any one of claims 1 to 10, **characterised in that** the band (4) has a length of 2 to 8 cm, preferably of 2.5 to 6 cm.

12. Implant according to any one of claims 1 to 11, **characterised in that** the band (4) has a width of 0.5 to 3 cm, preferably 0.7 to 2.5 cm, more preferably 1 to 2 cm.

13. Implant according to any one of claims 1 to 12, **characterised in that** the tube (7) has an inner diameter in between 1 to 4 cm, preferably in between 1.5 to 3.5 cm, more preferably in between 2 to 3 cm.

14. Implant according to any one of claims 1 to 13, **characterised in that** the tube (7) has a length of 5 to 40 cm, preferably of 10 to 30 cm, more preferably of 10 to 25 cm.

15. Implant according to any one of claims 1 to 14, **characterised in that** the band assembly with the inflatable balloon (3) enables reduction of blood perfusion in the vessel (11) in the range of 0 to 100%.

## Patentansprüche

1. Implantat zur Regulierung der Blutperfusion im Inneren eines Gefäßes (11), insbesondere innerhalb der Pfortader, umfassend eine aufblasbare Bandanordnung, einschließend:
a) ein nicht extendierbares Band (4), das ausgebildet ist, um lösbar um das Gefäß (11) herum fixiert zu werden, wobei das Band (4) ein hinteres Ende und ein vorderes Ende zur Aufnahme des hinteren Endes sowie eine lösbare Verschlussvorrichtung (6) umfasst, die das vordere und das hintere Ende des Bands (4) in einer gefäßumschließenden Position fixiert, und wobei das Band (4) mindestens einen aufblasbaren Ballon (3) einschließt, der an der inneren Seite des sich in einer gefäßumschließenden Position befindenden Bands (4) angeordnet ist, wobei sich der mindestens eine aufblasbare Ballon (3) über bis zu 60% des inneren Umfangs des sich in einer gefäßumschließenden Position befindenden Bands (4) erstreckt,
b) einen Injektionsanschluss (1) mit einer wieder verschließbaren Membran (5) oder einem Reservoir, das ein Fluid umfasst, und
c) einen Füllschlauch (2), der eine Fluidkommunikation zwischen dem Injektionsanschluss (1) oder dem Reservoir und dem mindestens einen aufblasbaren Ballon (3) der Bandanordnung bereitstellt, wobei an der inneren Seite der sich in der gefäßumschließenden Position befindenden Bandanordnung ein Mittel zur Messung des Blutflusses (14) im Inneren des Gefäßes (11) angeordnet ist und wobei zumindest ein Teil des Füllschlauchs (2) im Inneren eines Schlauchs (7) angeordnet ist, der ein proximales Ende und ein distales Ende (8) aufweist, wobei das distale Ende (8) des Schlauchs benachbart zu der Bandanordnung angeordnet ist und in der das Gefäß (11) umschließenden Position eine Form ähnlich einer das nicht extendierbare Band (4) umgebenden Manschette aufweist, wobei der Schlauch (7) Mittel zum Öffnen der Verschlussvorrichtung (6) umfasst, die einen Draht (9), bevorzugt einen Schneidedraht, umfassen, wobei der Draht (9) in einer Schlinge um die Verschlussvorrichtung (6) angeordnet ist, insbesondere in einer Schlinge um mindestens einen in der Verschlussvorrichtung (6) umfassten Zacken oder Haken (13), wobei die Verschlussvorrichtung (6) in dem vorderen Ende des Weiteren mindestens eine Kerbe (12) zur Aufnahme des Zackens (13) oder Hakens umfasst, wobei der Draht (9) im Inneren einer flexiblen, nicht extendierbaren Drahtführung (10) angeordnet ist, die auf dem Schlauch (7) fixiert ist.

2. Implantat nach Anspruch 1, **dadurch gekennzeichnet, dass** es sich bei dem Mittel zur Messung des Blutflusses (14) um ein Mittel zur Messung der Durchflussgeschwindigkeit des Typs Doppler und/oder um eine Doppler-Sonde zur Erfassung der Durchflusszeit handelt, das bzw. die stromabwärts des Bands vorgesehen ist.

3. Implantat nach Anspruch 1 oder 2, **dadurch gekennzeichnet, dass** die Mittel zur Messung des Blutflusses (14) gegenüber dem aufblasbaren Ballon (3) positioniert sind.

4. Implantat nach einem der Ansprüche 1 bis 3, **dadurch gekennzeichnet, dass** die Verschlussvorrichtung (6) mindestens einen Zacken (13) oder einen Haken, der jeweils auf einem Abschnitt des hinteren Endes des Bands ausgebildet ist, sowie mindestens eine Kerbe (12) in dem vorderen Ende zur Aufnahme des Zackens (13) oder des Hakens umfasst.

5. Implantat nach einem der Ansprüche 1 bis 4, **dadurch gekennzeichnet, dass** das Mittel zur Messung des Blutflusses (14) mit einem Transponder und/oder einer implantierten elektrischen Pumpe in Verbindung steht, um Fluid entweder aus dem Reservoir in den aufblasbaren Ballon (3) des Bands oder in die umgekehrte Richtung zu pumpen.

6. Implantat nach einem der Ansprüche 1 bis 5, **dadurch gekennzeichnet, dass** ein Mittel zur Messung des Drucks an der inneren Seite des sich in der gefäßumschließenden Position befindenden Bands (4) angeordnet ist.

7. Implantat nach einem der Ansprüche 1 bis 6, **dadurch gekennzeichnet, dass** das proximale Ende des Schlauchs (7) durch ein Mittel zum Verschließen, bevorzugt durch eine Membran, verschlossen ist.

8. Implantat nach Anspruch 7, **dadurch gekennzeichnet, dass** es sich bei dem Mittel zum Verschließen um einen selbstschließenden Stopfen aus einem Elastomer, bevorzugt aus Silikon oder Polyurethan, und mehr bevorzugt um einen wieder herausnehmbaren Silikon- oder Polyurethanstopfen handelt.

9. Implantat nach einem der Ansprüche 1 bis 8, **dadurch gekennzeichnet, dass** das Band (4), der Füllschlauch (2) und/oder der Schlauch (7) aus einem Elastomer, bevorzugt aus Silikon, ausgebildet sind.

10. Implantat nach einem der Ansprüche 1 bis 9, **dadurch gekennzeichnet, dass** der innere Durchmesser des sich in der gefäßumschließenden Position befindenden Bands (4) zwischen 0,8 und 3 cm, bevorzugt zwischen 1 und 2 cm, beträgt.

11. Implantat nach einem der Ansprüche 1 bis 10, **dadurch gekennzeichnet, dass** das Band (4) eine Länge von zwischen 2 und 8 cm, bevorzugt von zwischen 2,5 und 6 cm, aufweist.

12. Implantat nach einem der Ansprüche 1 bis 11, **dadurch gekennzeichnet, dass** dass Band eine Breite von zwischen 0,5 und 3 cm, bevorzugt von zwischen 0,7 und 2,5 cm, mehr bevorzugt von zwischen 1 und 2 cm, aufweist.

13. Implantat nach einem der Ansprüche 1 bis 12, **dadurch gekennzeichnet, dass** der Schlauch (7) einen Innendurchmesser von zwischen 1 und 4 cm, bevorzugt von zwischen 1,5 und 3,5 cm, mehr bevorzugt von zwischen 2 und 3 cm, aufweist.

14. Implantat nach einem der Ansprüche 1 bis 13, **dadurch gekennzeichnet, dass** der Schlauch (7) eine Länge von zwischen 5 und 40 cm, bevorzugt von zwischen 10 und 30 cm, mehr bevorzugt von zwischen 10 und 25 cm, aufweist.

15. Implantat nach einem der Ansprüche 1 bis 14, **dadurch gekennzeichnet, dass** die Bandanordnung mit dem aufblasbaren Ballon (3) eine Reduktion der Blutperfusion in dem Gefäß (11) in einem Bereich von zwischen 0 und 100% ermöglicht.

## Revendications

1. Implant pour réguler une perfusion sanguine à l'intérieur d'un vaisseau (11), en particulier la veine porte, comprenant un ensemble de bande gonflable, comprenant
a) une bande non extensible (4) configurée pour être fixée de manière amovible autour du vaisseau (11), dans lequel la bande (4) comprend une extrémité de queue et une extrémité de tête pour recevoir ladite extrémité de queue et des moyens de blocage amovible (6) fixant lesdites extrémités de tête et de queue de ladite bande (4) dans une position d'encerclement, et dans lequel ladite bande (4) comprend au moins un ballonnet gonflable (3) agencé au niveau du côté interne de la bande dans sa position d'encerclement, ledit au moins un ballonnet gonflable (3) s'étendant jusqu'à 60 % sur la circonférence interne de la bande (4) dans sa position d'encerclement,
b) un orifice d'injection (1) avec une membrane refermable (5) ou un réservoir comprenant un fluide, et
c) un tube de gonflage (2) fournissant la communication de fluide entre l'orifice d'injection (1) ou le réservoir et le au moins un ballonnet gonflable (3) de l'ensemble de bande, dans lequel, du côté interne de l'ensemble de bande dans sa position d'encerclement, des moyens pour mesurer l'écoulement de sang (14) à l'intérieur du vaisseau (11) sont agencés, et dans lequel au moins une partie du tube de gonflage (2) est agencée à l'intérieur d'un tube (7) ayant une extrémité proximale et une extrémité distale (8), l'extrémité distale (8) étant agencée de manière adjacente à l'ensemble de bande, ladite extrémité distale (8) du tube ayant une forme de manchette entourant la bande non extensible (4) dans sa position d'encerclement autour du vaisseau (11), dans lequel des moyens pour ouvrir les moyens de blocage (6) sont compris à l'intérieur dudit tube (7), qui comprennent un fil (9) adapté pour ouvrir lesdits moyens de blocage, de préférence un fil de coupe, dans lequel le fil (9) décrit une boucle autour des moyens de blocage (6), en particulier une boucle autour d'au moins une dent ou ardillon (13) d'une extrémité de queue de la bande (4) comprise à l'intérieur des moyens de blocage (6), comprenant en outre au moins une encoche (12) dans l'extrémité de tête pour mettre en prise ladite dent (13) ou ardillon, ledit fil (9) étant agencé à l'intérieur d'un guide-fil flexible non extensible (10) fixé sur le tube (7).

2. Implant selon la revendication 1, **caractérisé en ce que** les moyens de mesure d'écoulement (14) sont des moyens de mesure de vitesse de type Doppler et/ou une sonde Doppler de temps de transit placée en aval de la bande.

3. Implant selon la revendication 1 ou 2, **caractérisé en ce que** les moyens de mesure d'écoulement (14) sont positionnés à l'opposé du ballonnet gonflable (3).

4. Implant selon l'une quelconque des revendications 1 à 3, **caractérisé en ce que** les moyens de blocage (6) comprennent au moins une dent (13) ou un ardillon formé(e) sur une partie de l'extrémité de queue de la bande et au moins une encoche (12) dans l'extrémité de tête pour mettre en prise ladite dent (13) ou ardillon.

5. Implant selon l'une quelconque des revendications 1 à 4, **caractérisé en ce que** les moyens de mesure d'écoulement (14) sont raccordés à un transpondeur et/ou un implant de pompage électrique pour pomper le fluide du réservoir jusqu'au ballonnet gonflable (3) de la bande et vice-et-versa.

6. Implant selon l'une quelconque des revendications 1 à 5, **caractérisé en ce que** des moyens de mesure de pression sont agencés au niveau du côté interne de la bande (4) dans sa position d'encerclement.

7. Implant selon l'une quelconque des revendications 1 à 6, **caractérisé en ce que** l'extrémité proximale du tube (7) est scellée avec des moyens de scellement, de préférence une membrane.

8. Implant selon la revendication 7, **caractérisé en ce que** les moyens de scellement sont un élastomère d'auto-scellement, de préférence un bouchon en silicone ou polyuréthane, de manière plus préférée un bouchon en silicone ou polyuréthane amovible.

9. Implant selon l'une quelconque des revendications 1 à 8, **caractérisé en ce que** la bande (4), le tube de gonflage (2) et/ou le tube (7) sont réalisés à partir d'un élastomère, de préférence à partir de silicone.

10. Implant selon l'une quelconque des revendications 1 à 9, **caractérisé en ce que** le diamètre interne de la bande (4) dans sa position d'encerclement est compris entre 0,8 et 3 cm, de préférence 1 à 2 cm.

11. Implant selon l'une quelconque des revendications 1 à 10, **caractérisé en ce que** la bande (4) a une longueur de 2 à 8 cm, de préférence de 2,5 à 6 cm.

12. Implant selon l'une quelconque des revendications 1 à 11, **caractérisé en ce que** la bande (4) a une largeur de 0,5 à 3 cm, de préférence de 0,7 à 2,5 cm, de manière plus préférée de 1 à 2 cm.

13. Implant selon l'une quelconque des revendications 1 à 12, **caractérisé en ce que** le tube (7) a un diamètre interne compris entre 1 et 4 cm, de préférence compris entre 1,5 et 3,5 cm, de manière plus préférée compris entre 2 et 3 cm.

14. Implant selon l'une quelconque des revendications 1 à 13, **caractérisé en ce que** le tube (7) a une longueur de 5 à 40 cm, de préférence de 10 à 30 cm, de manière plus préférée de 10 à 25 cm.

15. Implant selon l'une quelconque des revendications 1 à 14, **caractérisé en ce que** l'ensemble de bande avec le ballonnet gonflable (3) permet une réduction de la perfusion sanguine dans le vaisseau (11) de l'ordre de 0 à 100 %.
